# EUROPEAN PATENT SPECIFICATION

(11) **EP 3 222 227 B1**
(45) Date of publication and mention of the grant of the patent: **14.11.2018**
(21) Application number: 16203576.0
(22) Date of filing: 13.12.2016
(51) Int. Cl.: A61B 17/3207, A61M 25/10, A61B 17/22, A61M 29/02, A61B 17/00

(54) **MEDICAL DEVICE**
MEDIZINISCHE VORRICHTUNG
DISPOSITIF MÉDICAL

(30) Priority: 25.03.2016 JP 2016062405
(43) Date of publication of application: 27.09.2017
(73) Proprietor: ASAHI INTECC CO., LTD., Nagoya-shi, Aichi 463-0024 (JP)
(72) Inventor: Enami, Yukiko, Nagoya-shi, Aichi 463-0024 (JP)
(74) Representative: Winter, Brandl, Fürniss, Hübner, Röss, Kaiser, Polte - Partnerschaft mbB

(56) References cited:
- WO-A1-98/50101
- WO-A1-2014/063039
- JP-A- 2015 173 913
- US-B2- 8 992 553

## Description

### [Field]

The present invention relates to a medical device comprising a mesh member capable of expanding and contracting (expandable and contractible) radially.

### Background

Conventionally, a stenosis or stricture formed in a blood vessel or in a digestive organ is treated by a well-known method (PTCA: percutaneous transluminal coronary angioplasty), by which method the stenosis or stricture is dilated with a medical device such as a balloon catheter.

Such a medical device generally has an expandable-and-contractible member capable of radially expanding and radially contracting (a balloon or a mesh member), and the expandable-and-contractible member (a balloon or a mesh member) is made to expand radially to dilate the stenosis or stricture.

When the stenosis or stricture has been highly calcified, however, it is difficult to dilate it with a balloon-type expandable-and-contractible member. So, for a calcified stenosis or stricture, a medical device comprising a mesh-type expandable-and-contractible member is often used (see Patent Literature 1 below, for example).

The medical device (an angioplasty catheter) of Patent Literature 1 comprises a mesh member made from a scoring element. The mesh member has, at an end thereof, a lining portion covering the inner circumference of the end (a ring-shaped member) as well as a wrapping portion covering the outer circumference of the end (a covering tube).

In this medical device, the fixation of the lining portion (the ring-shaped member) and the wrapping portion (the covering tube) to the end of the mesh member is firm and secure. Because of this firm fixation, upon radial expansion or radial contraction of the mesh member, the lining portion (the ring-shaped member) and the wrapping portion (the covering tube) interfere with the movement of the wire that constitute the mesh member (specifically, they interfere with changes in the angles made by the wire that constitute the mesh member against the axis), which is a problem. As a consequence, high stress is concentrated at the end of the mesh member, and the wires that constitute the mesh member are likely to break, which is also a problem. Especially when the mesh member is made to expand in a stenosis or stricture that is calcified and formed in a curving section of a blood vessel or a digestive organ, the mesh member cannot properly follow the curving contour of the blood vessel or the digestive organ and results in failed dilation of the stenosis or stricture or in breakage of the wire that constitute the mesh member.

### [Citation List]

### [Patent Literature]

Patent Literature 1: Japanese Patent Application Publication No. 2015-173913 (JP 2015-173913 A)

### [Summary]

### [Technical Problem]

The present invention is devised based on the above circumstances, and an object of the present invention is to provide a medical device in which movement of wire that constitute a mesh member of the medical device is not likely to be interfered with, concentration of stress at an end of the mesh member upon radial expansion or radial contraction of the mesh member is reduced, and consequently the wire that constitute the mesh member are not likely to break.

### [Solution to Problem]

This object is achieved in the following way.
A medical device that is disclosed in Embodiment 1 of the present invention comprises a mesh member capable of radially expanding and radially contracting, the mesh member being woven from a first wire and a second wire; and a cylindrical member comprising a wrapping portion and a lining portion, the wrapping portion covering an outer circumference of at least one end of the mesh member, the lining portion covering an inner circumference of the at least one end of the mesh member, wherein the wrapping portion and the lining portion are fixed to each other at fixation portions, the fixation portions being within spaces that are defined by the first wire and the second wire, and wherein neither the wrapping portion nor the lining portion is fixed to any of the first wire or the second wire.
Embodiment 2 of the present invention provides:
   the medical device according to embodiment 1, wherein the wrapping portion has a projected-and-depressed outer surface composed of depressed sections and projected sections, the depressed sections being formed at the fixation portions, the projected sections being formed where the wrapping portion and the lining portion are not fixed to each other.
Embodiment 3 of the present invention provides:
   the medical device according to embodiment 2, further comprises a shaft fixed to the projected-and-depressed outer surface of the wrapping portion and extending beyond the cylindrical member toward a proximal end, wherein the shaft is fixed to the cylindrical member at the depressed sections.
Embodiment 4 of the present invention provides:
   the medical device according to embodiment 1 to embodiment 3 further comprises a second cylindrical member comprising a second wrapping portion and a second lining portion, the second wrapping portion covering an outer circumference of the other end of the mesh member which are different from the one end of the mesh member, the second lining portion covering an inner circumference of the other end of the mesh member, wherein the second wrapping portion and the second lining portion are fixed to each other at second fixation portions, the second fixation portions being within spaces that are defined by the first wire and the second wire, and wherein neither the second wrapping portion nor the second lining portion is fixed to any of the first wire or the second wire.

### [Advantageous Effects of Invention]

The invention is defined in claim 1. A medical device according to embodiment 1 of the present invention has a configuration where a cylindrical member comprises a wrapping portion and a lining portion, where the wrapping portion and the lining portion are fixed to each other at fixation portions, with the fixation portions being within spaces that are defined by first wire and second wire, the first wire and the second wire constituting the mesh member, and where neither the wrapping portion nor the lining portion is fixed to any of the first wire or the second wire. Because of this configuration, upon radial expansion or radial contraction of the mesh member, movement of the first wire and the second wire is not likely to be interfered with by the cylindrical member, and consequently the concentration of stress at the end of the mesh member is reduced, so that the likelihood of breakage of the first wire or the second wire that constitute the mesh member is reduced. In addition, because this configuration gives the first wire and the second wire a certain degree of freedom in their movement, the mesh member, when made to expand in a stenosis or stricture that is formed in a curving section of a blood vessel or a digestive organ, can follow the curving contour of the blood vessel or the digestive organ and can dilate the stenosis or stricture more reliably than prior art does.

A medical device according to embodiment 2 of the present invention has a configuration where the wrapping portion of the cylindrical member has a projected-and-depressed outer surface composed of depressed sections and projected sections, with the depressed sections being formed at the fixation portions, and the projected sections being formed where the wrapping portion and the lining portion are not fixed to each other. This configuration, upon the insertion of the medical device into a blood vessel or a digestive organ, can reduce frictional resistance (area of contact) between the wrapping portion of the cylindrical member and the wall of the blood vessel or the wall of the digestive organ, allowing easy delivery of the medical device to the site of the stenosis or stricture. This configuration is preferable particularly for a stenosis or stricture formed in a sharply-curving peripheral section, where the wrapping portion of the cylindrical member is less likely to be caught on the wall of the blood vessel or the wall of the digestive organ.

A medical device according to embodiment 3 of the present invention comprises a shaft being fixed to the projected-and-depressed outer surface of the wrapping portion and extending beyond the cylindrical member toward a proximal end. The shaft is fixed to the cylindrical member at the depressed sections that are formed at the fixation portions. The anchoring effect can enhance the fixation between the shaft and the cylindrical member, reducing the possibility of the shaft coming off the cylindrical member.

A medical device according to embodiment 4 of the present invention further comprises a second cylindrical member on the other end of the mesh member, with the other end of the mesh member being different from one end of the mesh member, the second cylindrical member comprising a second wrapping portion and a second lining portion, the second wrapping portion and the second lining portion being fixed to each other at second fixation portions, the second fixation portions being within spaces that are defined by the first wire and the second wire, and neither the second wrapping portion nor the second lining portion being fixed to any of the first wire or the second wire. Because of this configuration, upon expansion or contraction of the mesh member, movement of the first wire and the second wire is not likely to be interfered with, and consequently the concentration of stress at either end of the mesh member is reduced, so that the likelihood of breakage of the first wire or the second wire that constitute the mesh member is further reduced.

### [Brief Description of Drawings]

FIG. 1 is an overall view of a medical device according to a present embodiment, in which the mesh member is radially contracted.
FIG. 2 is an overall view of a medical device according to a present embodiment, in which the mesh member is radially expanded.
FIG. 3 is an expanded view of the part A in FIG. 1.
FIG. 4 is a perspective view of FIG. 3.
FIG. 5 is a sectional view taken from line D-D of FIG. 3.
FIG. 6 shows part of a medical device according to a second embodiment, and is a variation of FIG. 5.
FIG. 7 shows part of a medical device according to a third embodiment, and is a variation of FIG. 6.
FIG. 8 shows part of a medical device according to a fourth embodiment, and is a variation of FIG. 3.
FIG. 9 is a sectional view taken from line E-E of FIG. 8.
FIG. 10 is an overall view of a medical device according to a fifth embodiment.
FIG. 11 is a sectional view taken from line F-F of FIG. 10.

### [Description of Embodiments]

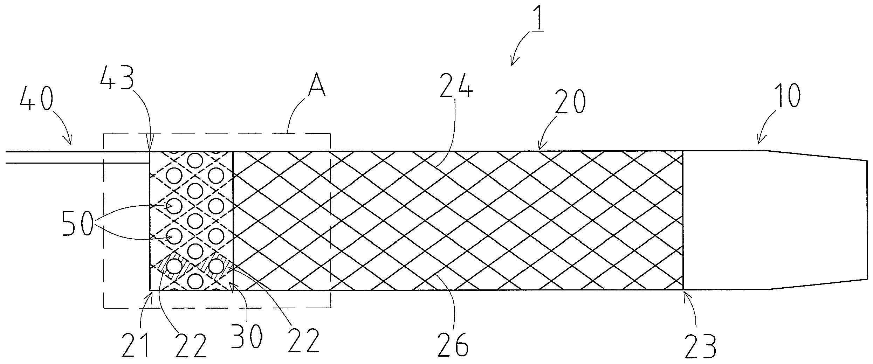

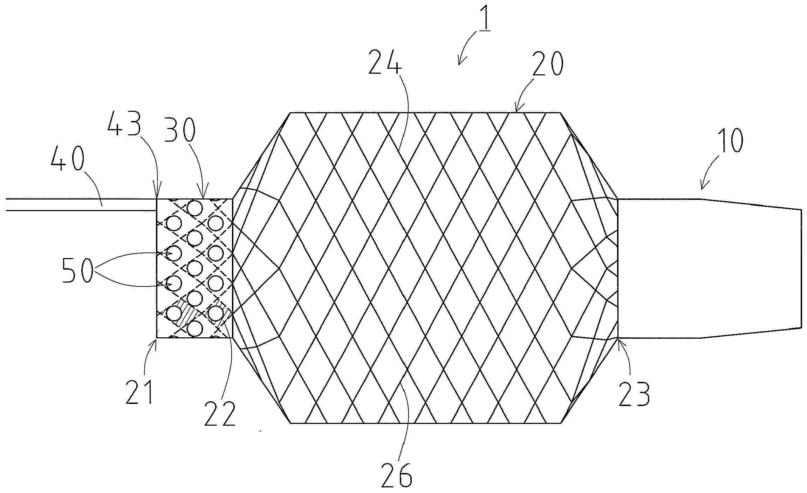

A medical device 1 according to a first embodiment is described below with reference to FIG. 1 to FIG. 5. In FIG. 1 to FIG. 4, the distal-end side (the front-end side) to be inserted into the body is shown to the right hand side, and the proximal-end side (the rear-end side, the base-end side) to be manipulated by a handler such as a doctor is shown to the left hand side. FIG. 1 shows a mesh member 20 radially contracted, and FIG. 2 shows the mesh member 20 radially expanded.

The medical device 1 is, for example, a therapeutic device used for dilating a stenosis or stricture for curative purposes. As shown in FIG. 1 and FIG. 2, the medical device 1 essentially comprises a distal tip 10, a mesh member 20 capable of radially expanding and radially contracting, a cylindrical member 30, and a shaft 40.

The mesh member 20 has a mesh configuration (a net configuration) woven at least from a first wire 24 and a second wire 26. The first wire 24 and the second wire 26 are made of a Co-Cr alloy with high tensile strength in this embodiment, but the material is not limited thereto. For example, the first wire 24 and the second wire 26 may be made of a metal material such as stainless steel, W, Pt, a Pt-Ni alloy, a Ni-Ti alloy, or a Cu-Al-Ni alloy or a resin material such as polyester, polyurethane, polyolefin, polytetrafluoroethylene, or a silicon resin. The material of the first wire 24 may be different from the material of the second wire 26.

Preferably, at least one of the first wire 24 and the second wire 26 is made of a radiopaque material (tungsten, for example) because, in that case, imaging allows the handler to see exactly where the mesh member 20 is and consequently allows the handler to easily place the mesh member 20 in a stenosis or stricture.

The distal end 23 of the mesh member 20 has a resin distal tip 10 fixed thereto. In this embodiment, the distal tip 10 is made of polyurethane.

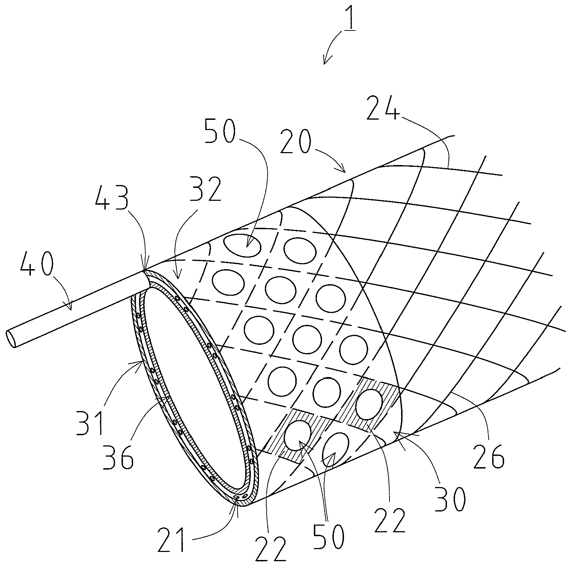

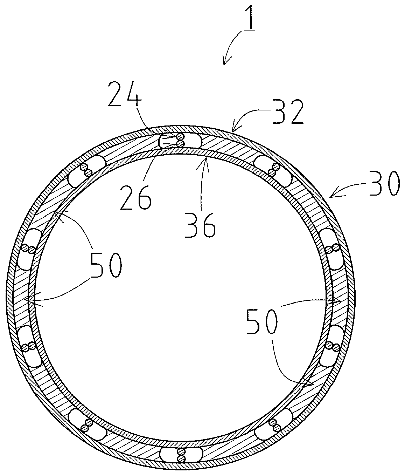

The proximal end 21 of the mesh member 20 has the cylindrical member 30 attached thereto. As shown in FIG. 4 and FIG. 5, the cylindrical member 30 has a wrapping portion 32 covering the outer circumference of the proximal end 21 of the mesh member 20 as well as a lining portion 36 covering the inner circumference of the proximal end 21 of the mesh member 20. Therefore, the outer circumference and the inner circumference of the proximal end 21 of the mesh member 20 are covered with the wrapping portion 32 and the lining portion 36 of the cylindrical member 30.

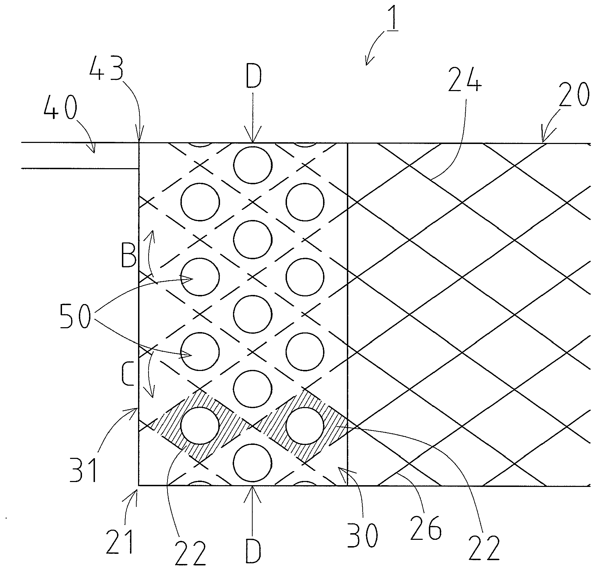

As shown in FIG. 3 and FIG. 4, to the proximal end 31 of the cylindrical member 30, the distal end 43 of the shaft 40 is fixed. The shaft 40 is a thin, cylinder-like metal wire (stainless steel), extending from the proximal end 31 of the cylindrical member 30 in the longitudinal direction toward the proximal-end side.

The handler inserts the medical device 1 into a blood vessel or a digestive organ and manipulates the shaft 40 in the longitudinal direction to deliver the medical device 1 to the site of a stenosis or stricture. During this procedure, the mesh member 20 is radially contracted (see FIG. 1). Subsequently, the handler inserts a balloon catheter into the interior of the mesh member 20 of the medical device 1 and then makes the balloon of the balloon catheter radially expand so as to radially expand the mesh member 20 (see FIG. 2). By pressing the mesh member 20 against the stenosis or stricture, the handler can dilate the stenosis or stricture.

In FIG. 2, the balloon catheter inserted in the interior of the mesh member 20 is omitted for ease of understanding. The balloon catheter used in the medical device 1 can be a known balloon catheter.

In the medical device 1 according to this embodiment, the wrapping portion 32 and the lining portion 36 are fixed to each other at fixation portions 50, with the fixation portions 50 being within spaces 22 on the circumference that are defined by the first wire 24 and the second wire 26, and neither the wrapping portion 32 nor the lining portion 36 is fixed to any of the first wire 24 or the second wire 26 that constitute the mesh member 20 (see FIG. 4 and FIG. 5). The wrapping portion 32 and the lining portion 36 of the cylindrical member 30 are made of stainless steel and are fixed to each other within the spaces 22 with an adhesive in this embodiment. However, the method of providing the fixation portions 50 is not particularly limited.

Because of this configuration, upon radial expansion (in other words, upon a shift from the state in FIG. 1 to the state in FIG. 2) or radial contraction (in other words, upon a shift from the state in FIG. 2 to the state in FIG. 1) of the mesh member 20, movement of the first wire 24 or the second wire 26 is not likely to be interfered with by the cylindrical member 30, and consequently the concentration of stress at the end 21 of the mesh member 20 is reduced, so that the likelihood of breakage of the first wire 24 or the second wire 26 that constitute the mesh member 20 is reduced. As shown in FIG. 3, upon expansion of the mesh member 20, the first wire 24 can move toward the direction B and the second wire 26 can move toward the direction C. As described here, because the first wire 24 and the second wire 26 are not fixed to the cylindrical member 30, the first wire 24 and the second wire 26 can move with a certain degree of freedom. As a result, the mesh member 20, when made to expand in a stenosis or stricture that is formed in a curving section of a blood vessel or a digestive organ, can follow the curving contour of the blood vessel or the digestive organ and can dilate the stenosis or stricture more reliably than prior art does.

The degree of freedom in the movement of the first wire 24 and the second wire 26 can be designed, as appropriate, by changing the size, shape, and number of the fixation portions 50 within the spaces 22.

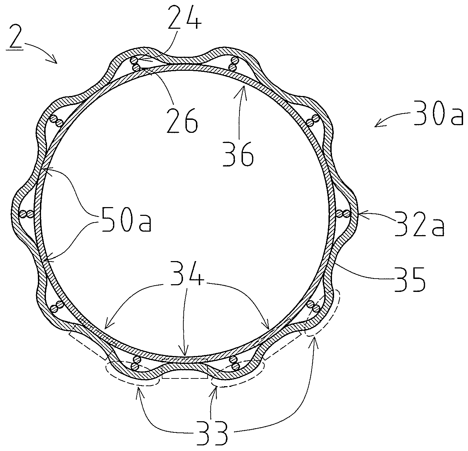

Next, a medical device 2 according to a second embodiment is described below with reference to FIG. 6. FIG. 6 is a variation of FIG. 5. The description of the medical device 2 below only includes its differences from the medical device 1. In the medical device 2, a wrapping portion 32a of a cylindrical member 30a has a projected-and-depressed outer surface 35. More specifically, the projected-and-depressed outer surface 35 of the wrapping portion 32a is composed of depressed sections 34 and projected sections 33, the depressed sections 34 being formed within the spaces 22 at fixation portions 50a at which the wrapping portion 32a and the lining portion 36 are fixed to each other, the projected sections 33 being formed where the wrapping portion 32a and the lining portion 36 are not fixed to each other.

This configuration, upon insertion of the medical device 2 into a blood vessel or a digestive organ, can reduce frictional resistance (area of contact) between the wrapping portion 32a of the cylindrical member 30a and the wall of the blood vessel or the wall of the digestive organ, allowing easy delivery of the medical device 2 to the site of the stenosis or stricture. This configuration is preferable particularly for a stenosis or stricture formed in a sharply-curving peripheral section, where the wrapping portion 32a of the cylindrical member 30a is less likely to be caught on the wall of the blood vessel or the wall of the digestive organ.

The method of forming the fixation portions 50a is not particularly limited. For example, the wrapping portion 32a with the projected-and-depressed outer surface 35 may be prepared in advance, followed by the formation of the fixation portions 50a by using the wrapping portion 32a, the lining portion 36, and an adhesive. Alternatively, a laser may be applied from outside to the spaces 22 in the cylindrical member 30 of the medical device 1. By this laser application, the fixation portions 50a at which the wrapping portion 32a and the lining portion 36 are fixed to each other are formed, and, at the same time, the projected-and-depressed outer surface 35 of the wrapping portion 32a is formed.

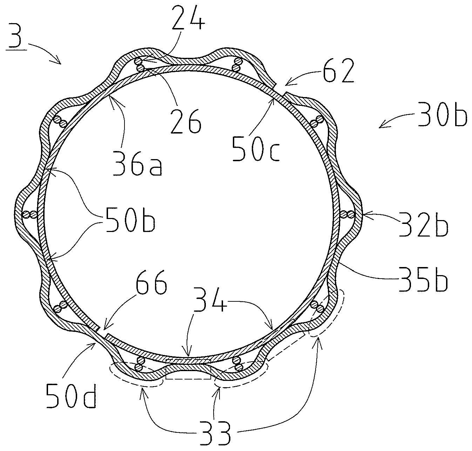

Next, a medical device 3 according to a third embodiment is described below with reference to FIG. 7. FIG. 7 is a variation of FIG. 6. The description of the medical device 3 below only includes its differences from the medical device 2. In the medical device 3, similar to the wrapping portion 32a of the medical device 2, a wrapping portion 32b of a cylindrical member 30b has a projected-and-depressed outer surface 35b composed of depressed sections 34 the projected sections 33, and it also has a space 62. In addition, a lining portion 36a of the cylindrical member 30b has a space 66. At the space 62, the wrapping portion 32b and the lining portion 36a are fixed to each other at a fixation portion 50c, and at the space 66, the wrapping portion 32b and the lining portion 36a are fixed to each other at a fixation portion 50d.

In the configuration of the cylindrical member 30b of the medical device 3, the lining portion 36a, which is a rolled stainless steel tabular member covering the inner circumference of the proximal end 21 of the mesh member 20, is fixed to the wrapping portion 32b, which is a rolled stainless steel tabular member covering the outer circumference of the proximal end 21 of the mesh member 20, at the fixation portions 50b, 50c, and 50d. In FIG. 7, the two ends of the tabular member that are to be made into the lining portion 36a are spaced from each other in order to form the space 66 in the lining portion 36a, while the two ends of the tabular member that are to be made into the wrapping portion 32b are spaced from each other in order to form the space 62 in the wrapping portion 32b. Due to this configuration of the cylindrical member 30b, there is no need to provide the cylindrical lining portion 36 or the cylindrical wrapping portion 32 or 32a as required in the cylindrical member 30 or 30a, but instead, the wrapping portion 32b can be formed into a cylindrical shape by fixation of the lining portion 36a and the wrapping portion 32b to each other at the fixation portion 50c. In the same manner, the lining portion 36a can be formed into a cylindrical shape by fixation of the lining portion 36a and the wrapping portion 32b to each other at the fixation portion 50d.

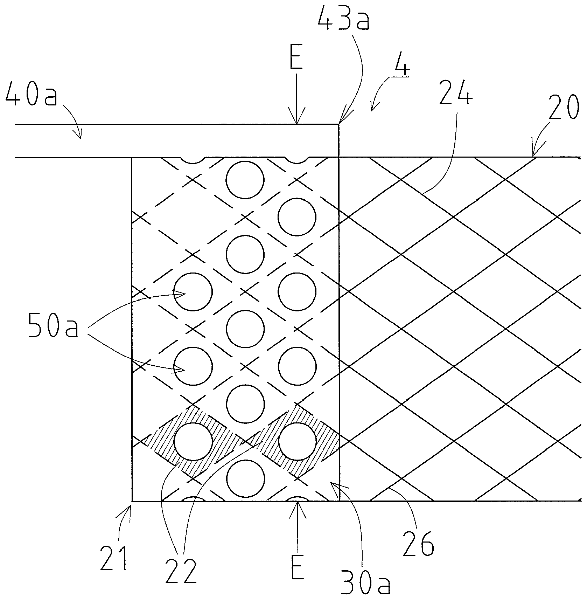

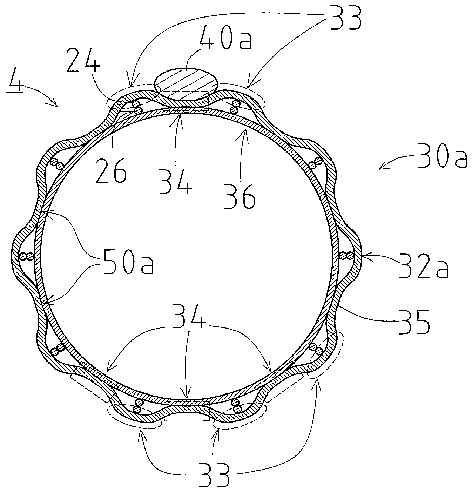

Next, a medical device 4 according to a fourth embodiment is described below reference to FIG. 8 and FIG. 9. FIG. 8 is a variation of FIG. 3. In FIG. 8, the distal-end side (the front-end side) to be inserted into the body is shown to the right hand side, and the proximal-end side (the rear-end side, the base-end side) to be manipulated by a handler such as a doctor is shown to the left hand side.

The description of the medical device 4 below only includes its differences from the medical device 2. As shown in FIG. 8, the distal end 43a of the shaft 40a of the medical device 4 extends to the distal-end side of the cylindrical member 30a and is fixed to the outer surface 35 of the wrapping portion 32a of the cylindrical member 30a. The shaft 40a extends in the longitudinal direction as it covers the fixation portions 50a at which the wrapping portion 32a and the lining portion 36 of the cylindrical member 30a are fixed to each other, which is described above for the medical device 2 according to a second embodiment. As shown in FIG. 9, the shaft 40a is fixed to the cylindrical member 30a at least in part, namely at the depressed sections 34 of the wrapping portion 32a.

Because the shaft 40a is thus buried at least in part in the depressed sections 34 of the fixation portions 50a, the anchoring effect can enhance the fixation between the shaft 40a and the cylindrical member 30a, reducing the possibility of the shaft 40a coming off of the cylindrical member 30a.

As shown in FIG. 9, the cross-sectional profile of the shaft 40a is elliptical so that it can fit into the depressed sections 34 of the outer surface 35 of the wrapping portion 32a of the cylindrical member 30a. This shape can increase the area at which the shaft 40a and the cylindrical member 30a are fixed to each other, and, as a result, can further enhance the fixation between the shaft 40a and the cylindrical member 30a.

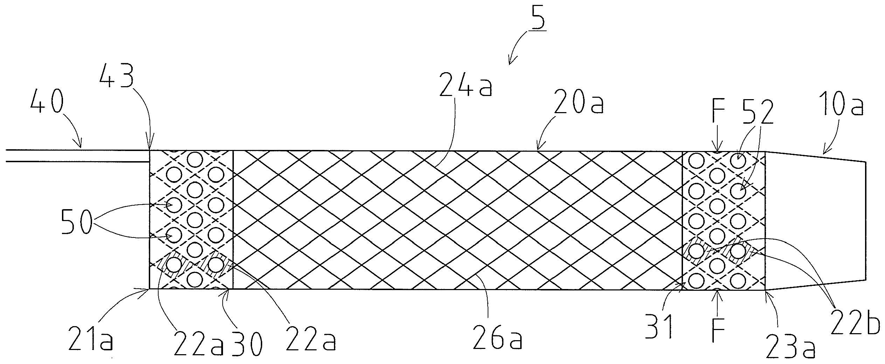

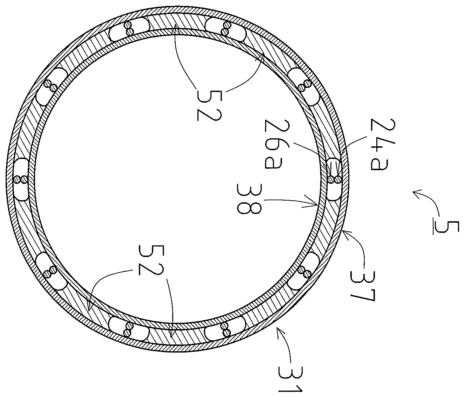

Lastly, a medical device 5 according to a fifth embodiment is described below with reference to FIG. 10 and FIG. 11. In FIG. 10, the distal-end side (the front-end side) to be inserted into the body is shown to the right hand side, and the proximal-end side (the rear-end side, the base-end side) to be manipulated by a handler such as a doctor is shown to the left hand side.

The description of the medical device 5 below only includes its differences from the medical device 1. As shown in FIG. 10, the proximal end 21a of the mesh member 20a has the cylindrical member 30 attached thereto, and the distal end 23a of the mesh member 20a has a second cylindrical member 31 attached thereto. As shown in FIG. 11, similarly to the cylindrical member 30, the second cylindrical member 31 has a second wrapping portion 37 covering the outer circumference of the distal end 23a of the mesh member 20a as well as a second lining portion 38 covering the inner circumference of the distal end 23a of the mesh member 20a. Therefore, the outer circumference and the inner circumference of the distal end 21a of the mesh member 20a are covered with the wrapping portion 32 and the lining portion 36 of the cylindrical member 30, and the outer circumference and the inner circumference of the distal end 23a of the mesh member 20a are covered with the second wrapping portion 37 and the second lining portion 38 of the second cylindrical member 31.

The distal end of the second cylindrical member 31 has a resin distal tip 10a fixed thereto.

In the medical device 5, the second wrapping portion 37 and the second lining portion 38 are fixed to each other at second fixation portions 52, the second fixation portions 52 being within spaces 22b on the circumference that are defined by first wire 24a and second wire 26a, and neither the second wrapping portion 37 nor the second lining portion 38 is fixed to any of the first wire 24a and the second wire 26a that constitute the mesh member 20a (see FIG. 11). As in the case of the cylindrical member 30, the second wrapping portion 37 and the second lining portion 38 of the second cylindrical member 31 are made of stainless steel and are fixed to each other within the spaces 22b with an adhesive. However, the method of providing the second fixation portions 52 is not particularly limited.

Because of this configuration, upon radial expansion or radial contraction of the mesh member 20a, movement of the first wire 24a or the second wire 26a is not likely to be interfered with by the cylindrical member 30 or the second cylindrical member 31, and consequently the concentration of stress at either end 21a or 23a of the mesh member 20a is reduced, so that the likelihood of breakage of the first wire 24a or the second wire 26a that constitute the mesh member 20a is further reduced.

In the medical devices 1 to 5 described above, dilation of a stenosis or stricture is achieved by using the mesh member 20 or 20a alone. However, when the stenosis or stricture has been calcified and the expansion of the mesh member is not enough, the mesh member may have an incising member (a scoring member) attached on its outer circumference in the longitudinal direction. When attaching the incising member (the scoring member), attention should be paid so that it does not interfere with the movement of the first wire 24 or 24a or the second wire 26 or 26a upon radial expansion or radial contraction of the mesh member 20 or 20a.

In the medical devices 1 to 5 described above, the fixation portions 50, 50a, 50b, 50c, or 50d at which the wrapping portion 32, 32a, or 32b and the lining portion 36 or 36a are fixed to each other are within all the spaces 22, 22a, or 22b on the circumference that are defined by the first wire 24 or 24a and the second wire 26 or 26a. However, this configuration is not limitative. As long as the first wire 24 or 24a and the second wire 26 or 26a do not move out of position upon radial expansion or radial contraction of the mesh member 20 or 20a, the fixation portions 50, 50a, 50b, 50c, or 50d may be formed sporadically (infrequently) on the circumference, or the fixation portions 50, 50a, 50b, 50c, or 50d may be formed in a spiral arrangement.

The material of the wrapping portion 32, 32a, or 32b and the lining portion 36 or 36a of the cylindrical member 30, 30a, or 30b is not limited to stainless steel. Preferably, the material thereof is a radiopaque material (tungsten, for example) because, in that case, imaging allows the handler to see exactly where the proximal end of the mesh member 20 is and consequently to easily place the mesh member 20 or 20a in a stenosis or stricture. Similarly, the material of the second wrapping portion 37 and the second lining portion of the second cylindrical member 31 is not limited to stainless steel. Preferably, the material thereof is a radiopaque material (tungsten, for example) because, in that case, imaging allows the handler to see exactly where the distal end of the mesh member 20a is and consequently allows the handler to easily place the mesh member 20a in a stenosis or stricture.

The cross-sectional profile of the cylindrical member 30, 30a, or 30b and the second cylindrical member 31 is described above as circular, but it is not limitative. The cross-sectional profile thereof may be elliptical or polygonal.

### [Reference Signs List]

- 1, 2, 3, 4, 5:: Medical device
- 10, 10a:: Distal tip
- 20, 20a:: Mesh member
- 22, 22a, 22b:: Space
- 24, 24a:: First elemental thread
- 26, 26a:: Second elemental thread
- 30, 30a, 30b:: Cylindrical member
- 31:: Second cylindrical member
- 32, 32a, 32b:: Wrapping portion
- 33:: Projected section
- 34:: Depressed section
- 35:: Projected-and-depressed outer surface
- 36, 36a:: Lining portion
- 37:: Second wrapping portion
- 38:: Second lining portion
- 40, 40a:: Shaft
- 50, 50a, 50b, 50c, 50d:: Fixation portion
- 52:: Second fixation portion
- 62,: 66: Space

## Claims

1. A medical device (1, 2, 3, 4, 5) comprising:
a tubular mesh member (20, 20a) capable of radially expanding and radially contracting, the mesh member (20, 20a) being woven from a first wire (24, 24a) and a second wire (26, 26a); and
a cylindrical member (30, 30a, 30b) comprising a wrapping portion (32, 32a, 32b) and a lining portion (36, 36a), the wrapping portion (32, 32a, 32b) covering an outer circumference of at least one end (21, 21a) of the mesh member, the lining portion (36, 36a) covering an inner circumference of the at least one end (21, 21a) of the mesh member (20, 20a),
wherein the wrapping portion (32, 32a, 32b) and the lining portion (36, 36a) are fixed to each other at fixation portions (50, 50a, 50b, 50c, 50d), the fixation portions (50, 50a, 50b, 50c, 50d) being within spaces (22, 22a, 22b) that are defined by the first wire (24, 24a) and the second wire (26, 26a), and
wherein neither the wrapping portion (32, 32a, 32b) nor the lining portion (36, 36a) is fixed to any of the first wire (24, 24a) or the second wire (26, 26a).

2. The medical device (2, 3, 4) according to claim 1, wherein the wrapping portion (32a, 32b) has a projected-and-depressed outer surface (35, 35b) composed of depressed sections (34) and projected sections (33), the depressed sections (34) being formed at the fixation portions (50a, 50b, 50c, 50d), the projected sections (33) being formed where the wrapping portion (32a, 32b) and the lining portion (36, 36a) are not fixed to each other.

3. The medical device (4) according to claim 2, having a proximal end and a distal end and further comprising:
a shaft (40a) fixed to the projected-and-depressed outer surface (35) of the wrapping portion (32a) and extending beyond the cylindrical member (30a) toward a proximal end,
wherein the shaft (40a) is fixed to the cylindrical member (30a) at the depressed sections (34).

4. The medical device (5) according to any one of claims 1 to 3, further comprising:
a second cylindrical member (31) comprising a second wrapping portion (37) and a second lining portion (38), the second wrapping portion (37) covering an outer circumference of the other end (23a) of the mesh member (20a) which are different from the one end (21a) of the mesh member (20a), the second lining portion (38) covering an inner circumference of the other end (23a) of the mesh member (20a),
wherein the second wrapping portion (37) and the second lining portion (38) are fixed to each other at second fixation portions (52), the second fixation portions (52) being within spaces (22b) that are defined by the first wire (24a) and the second wire (26a), and
wherein neither the second wrapping portion (37) nor the second lining portion (38) is fixed to any of the first wire (24a) or the second wire (26a).

5. The medical device (3) according to claim 2, wherein at least of the wrapping portion (32b) and the lining portion (36a) has a space (62, 66) at the depressed sections (34).

## Patentansprüche

1. Medizinische Vorrichtung (1, 2, 3, 4, 5) mit:
einem radial dehn- und zusammenziehfähigen röhrenförmigen Gitterelement (20, 20a), das aus einem ersten Draht (24, 24a) und einem zweiten Draht (26, 26a) gewebt ist; und
einem zylindrischen Element (30, 30a, 30b), das eine Ummantelung (32, 32a, 32b) und eine Auskleidung (36, 36a) aufweist, wobei die Ummantelung (32, 32a, 32b) den Außenumfang wenigstens eines Endes (21, 21a) des Gitterelements und die Auskleidung (36, 36a) den Innenumfang des wenigstens einen Endes (21, 21a) des Gitterelements (20, 20a) bedeckt,
wobei die Ummantelung (32, 32a, 32b) und die Auskleidung (36, 36a) aneinander an Befestigungsstellen (50, 50a, 50b, 50c, 50d) befestigt sind, die in durch den ersten Draht (24, 24a) und den zweiten Draht (26, 26a) definierten Räumen (22, 22a, 22b) liegen, und
wobei weder die Ummantelung (32, 32a, 32b) noch die Auskleidung (36, 36a) an dem ersten Draht (24, 24a) oder zweiten Draht (26, 26a) befestigt ist.

2. Medizinische Vorrichtung (2, 3, 4) nach Anspruch 1, wobei die Ummantelung (32a, 32b) eine aus Vertiefungen (34) und Vorsprüngen (33) gebildete unebene Außenfläche (35, 35b) hat, wobei die Vertiefungen (34) an den Befestigungsstellen (50a, 50b, 50c, 50d) und die Vorsprünge (33) an Stellen ausgebildet sind, an denen die Ummantelung (32a, 32b) und die Auskleidung (36, 36a) nicht aneinander befestigt sind.

3. Medizinische Vorrichtung (4) nach Anspruch 2, mit einem proximalen Ende und einem distalen Ende und weiter mit:
einem an der unebenen Außenfläche (35) der Ummantelung (32a) befestigten Schaft (40a), der sich über das zylindrische Element (30a) hinaus in Richtung des proximalen Endes erstreckt,
wobei der Schaft (40a) in den Vertiefungen (34) an dem zylindrischen Element (30a) befestigt ist.

4. Medizinische Vorrichtung (5) nach einem der Ansprüche 1 bis 3, weiter mit:
einem zweiten zylindrischen Element (31), das eine zweite Ummantelung (37) und eine zweite Auskleidung (38) aufweist, wobei die zweite Ummantelung (37) den Außenumfang des von dem einen Ende (21a) des Gitterelements (20a) verschiedenen anderen Endes (23a) des Gitterelements (20a) und die zweite Auskleidung (38) den Innenumfang des anderen Endes (23a) des Gitterelements (20a) bedeckt,
wobei die zweite Ummantelung (37) und die zweite Auskleidung (38) aneinander an zweiten Befestigungsstellen (52) befestigt sind, die in durch den ersten Draht (24a) und den zweiten Draht (26a) definierten Räumen (22b) liegen, und
wobei weder die zweite Ummantelung (37) noch die zweite Auskleidung (38) an dem ersten Draht (24a) oder dem zweiten Draht (26a) befestigt ist.

5. Medizinische Vorrichtung (3) nach Anspruch 2, wobei die Ummantelung (32b) und/oder Auskleidung (36a) in den Vertiefungen (34) einen Raum (62, 66)hat.

## Revendications

1. Dispositif médical (1, 2, 3, 4, 5) comprenant :
un élément maillé tubulaire (20, 20a) susceptible de se dilater de façon radiale et de se contracter de façon radiale, l'élément maillé (20, 20a) étant tissé à partir d'un premier fil (24, 24a) et d'un second fil (26, 26a) ; et
un élément cylindrique (30, 30a, 30b) comprenant une portion d'enveloppement (32, 32a, 32b) et une portion de revêtement intérieur (36, 36a), la portion d'enveloppement (32, 32a, 32b) couvrant une circonférence extérieure d'au moins une première extrémité (21, 21a) de l'élément maillé, la portion de revêtement intérieur (36, 36a) couvrant une circonférence intérieure de l'au moins une première extrémité (21, 21a) de l'élément maillé (20, 20a),
dans lequel la portion d'enveloppement (32, 32a, 32b) et la portion de revêtement intérieur (36, 36a) sont fixées l'une à l'autre au niveau de portions de fixation (50, 50a, 50b, 50c, 50d), les portions de fixation (50, 50a, 50b, 50c, 50d) étant à l'intérieur d'espaces (22, 22a, 22b) qui sont définis par le premier fil (24, 24a) et le second fil (26, 26a), et
dans lequel ni la portion d'enveloppement (32, 32a, 32b) ni la portion de revêtement intérieur (36, 36a) ne sont fixées à l'un quelconque du premier fil (24, 24a) ou du second fil (26, 26a).

2. Dispositif médical (2, 3, 4) selon la revendication 1, dans lequel la portion d'enveloppement (32a, 32b) a une surface extérieure en saillie et enfoncée (35, 35b) composée de sections enfoncées (34) et de sections en saillie (33), les sections enfoncées (34) étant formées au niveau des portions de fixation (50a, 50b, 50c, 50d), les sections en saillie (33) étant formées là où la portion d'enveloppement (32a, 32b) et la portion de revêtement intérieur (36, 36a) ne sont pas fixées l'une à l'autre.

3. Dispositif médical (4) selon la revendication 2, ayant une extrémité proximale et une extrémité distale et comprenant en outre :
un arbre (40a) fixé à la surface extérieure en saillie-et-enfoncée (35) de la portion d'enveloppement (32a) et s'étendant au-delà de l'élément cylindrique (30a) vers une extrémité proximale,
dans lequel l'arbre (40a) est fixé à l'élément cylindrique (30a) au niveau des sections enfoncées (34).

4. Dispositif médical (5) selon l'une quelconque des revendications 1 à 3, comprenant en outre :
un second élément cylindrique (31) comprenant une seconde portion d'enveloppement (37) et une seconde portion de revêtement intérieur (38), la seconde portion d'enveloppement (37) couvrant une circonférence extérieure de l'autre extrémité (23a) de l'élément maillé (20a) qui est différente de la première extrémité (21a) de l'élément maillé (20a), la seconde portion de revêtement intérieur (38) couvrant une circonférence intérieure de l'autre extrémité (23a) de l'élément maillé (20a),
dans lequel la seconde portion d'enveloppement (37) et la seconde portion de revêtement intérieur (38) sont fixées l'une à l'autre au niveau de secondes portions de fixation (52), les secondes portions de fixation (52) étant à l'intérieur d'espaces (22b) qui sont définis par le premier fil (24a) et le second fil (26a), et
dans lequel ni la seconde portion d'enveloppement (37) ni la seconde portion de revêtement intérieur (38) ne sont fixées à l'un quelconque du premier fil (24a) ou du second fil (26a).

5. Dispositif médical (3) selon la revendication 2, dans lequel au moins une de la portion d'enveloppement (32b) et de la portion de revêtement intérieur (36a) a un espace (62, 66) au niveau des sections enfoncées (34).
